# EUROPEAN PATENT APPLICATION

(11) **EP 2 255 668 A2**
(43) Date of publication of application: **01.12.2010**
(21) Application number: 10176353.0
(22) Date of filing: 14.05.2002
(51) Int. Cl.: A23D 9/013, C11B 1/10, A61K 31/66, A61K 31/202, A23L 1/30, A23K 1/16

(54) **Production and Use of a Polar Lipid-Rich Fraction Containing Omega-3 and/or Omega-6 Highly Unsaturated Fatty Acids from Microbes, Genetically Modified Plant Seeds and Marine Organisms**

(30) Priority: 14.05.2001 US 290899 P
(62) Divisional of application: 02736881.0
(71) Applicant: MARTEK BIOSCIENCES CORPORATION, Columbia, MD 21045 (US)
(72) Inventor: The designation of the inventor has not yet been filed
(74) Representative: Mallalieu, Catherine Louise

(57) **Abstract**

The production and use, and in particular, the extraction, separation, synthesis and recovery of polar lipid-rich fractions containing eicosapentaenoic acid (EPA), docosahexaenoic acid (DHA), docosapentaenoic acid (DPA(n-3) or DPA(n-6)), arachidonic acid (ARA), and eicosatetraneonoic acid (C20:4n-3) from microorganisms, genetically modified seeds and marine organisms (including fish and squid) and their use in human food applications, animal feed, pharmaceutical applications and cosmetic applications.

## Description

### FIELD OF THE INVENTION

The present invention relates to the fields of production and use, and in particular, the extraction, separation, synthesis and recovery of polar lipid-rich fractions containing eicosapentaenoic acid (EPA), docosahexaenoic acid (DHA), docosapentaenoic acid (DPA(n-3) or DPA(n-6)), arachidonic acid (ARA), and eicosatetraenoic acid (C20:4n-3) from microorganisms, genetically modified seeds and marine organisms (including fish and squid) and their use in human food applications, animal feed, pharmaceutical applications and cosmetic applications.

### BACKGROUND OF THE INVENTION

Highly unsaturated fatty acids of the omega-6 and omega-3 series represent a special class of bioactive lipids in that they are important structurally in membranes in the body, but also participate directly and indirectly in communication between cells through the eicosanoid pathways and by their influence of these fatty acids on gene expression. Six of these fatty acids, eicosapentaenoic acid (EPA), docosahexaenoic acid (DHA), docosapentaenoic acid (DPA(n-3) or DPA(n-6)), arachidonic acid (ARA), and eicosatetraenoic acid (C20:4n-3) have been shown to be effective in preventing/treating cardiovascular disease, inflammatory disease, immune function imbalances, fertility and some of these fatty acids (ARA, DHA DPA(n-6) are important structurally in the brain and nervous system. Recent evidence indicates that some highly unsaturated fatty acids may be more bioavailable when supplied in a phospholipid form than in a triglyceride form. EPA, DHA, and ARA have historically been supplied to the nutritional supplement markets in the form of oil extracted from algae or fish. However recent evidence indicates that some polyunsaturated fatty acids may be more bioavailable in a phospholipid form rather than in a triglyceride form. This may be because of the bipolar nature of phospholipids, making them readily solubilizable in the gut and available for digestion and uptake. This same bipolar property of phospholipids additionally would make these fatty acids more functional in topical applications such as creams and lotions or more soluble in aqueous-based applications such as beverages because of there ability to participate in emulsification processes. We propose that there may be important advantages in supplying these omega-3 and omega-6 HUFAs in the form of phospholipids and improved processes for recovering polar lipids enriched in these fatty acids are also needed.

Examples of polar lipids include phospholipids (e.g. phosphatidylcholine, phosphatidylethanolamine, phosphatidylinositol, phosphatidylserine, phosphatidylglycerol, diphosphatidylglycerols), cephalins, sphingolipids (sphingomyelins and glycosphingolipids), and glycoglycerolipids. Phospholipids are composed of the following major structural units: fatty acids, glycerol, phosphoric acid, and amino alcohols. They are generally considered to be structural lipids, playing important roles in the structure of the membranes of plants, microbes and animals. Because of their chemical structure, polar lipids exhibit a bipolar nature, exhibiting solubility or partial solubility in both polar and non-polar solvents. The term polar lipid within the present description is not limited to natural polar lipids but also includes chemically modified polar lipids. Although the term oil has various meanings, as used herein, it will refer to the triacylglycerol fraction.

One of the important characteristics of polar lipids, and especially phospholipids, is that they commonly contain polyunsaturated fatty acids (PUFAs: fatty acids with 2 or more unsaturated bonds). In many plant, microbial and animal systems, they are especially enriched in the highly unsaturated fatty acids (HUFAs: fatty acids with 4 or more unsaturated bonds) of the omega-3 and omega-6 series. Although these highly unsaturated fatty acids are considered unstable in triacylglycerol form, they exhibit enhanced stability when incorporated in phospholipids.

The primary sources of commercial PUFA-rich phospholipids are soybeans and canola seeds. These biomaterials do not contain any appreciable amounts of highly unsaturated fatty acids unless they have been genetically modified. The phospholipids (commonly called lecithins) are routinely recovered from these oilseeds as a by-product of the vegetable oil extraction process. For example, in the production of soybean or canola oil, the beans (seeds) are first heat-treated and then crushed, ground, and/or flaked, followed by extraction with a non-polar solvent such as hexane. Hexane removes the triacylglycerol-rich fraction from the seeds together with a varying amount of polar lipids (lecithins). The extracted oil is then de-gummed (lecithin removal) either physically or chemically as a part of the normal oil refining process and the precipitated lecithins recovered. This process however has two disadvantages: (1) the seeds must be heat-treated before extraction with hexane, increasing the processing cost, and increasing undesirable oxidation reactions and denaturing the protein fraction, thereby decreasing its value as a by-product; and (2) the use of the non-polar solvents such as hexane also presents toxicity and flammability problems that must be dealt with.

The crude lecithin extracted in the "de-gumming" process can contain up to about 33% oil (triacylglycerols) along with sterols and glucosides. One preferred method for separating this oil from the crude lecithin is by extraction with acetone. The oil (triacylglycerols) is soluble in acetone and the lecithin is not. The acetone solution is separated from the precipitate (lecithin) by centrifugation and the precipitate dried under first a fluidized bed drier and then a vacuum drying oven to recover the residual acetone as the product is dried. Drying temperatures of 50-70°C are commonly used. The resulting dried lecithins contain approximately 2-4% by weight of oil (triacylglycerols). Process temperatures above 70°C can lead to thermal decomposition of the phospholipids. However, even at temperatures below 70°C the presence of acetone leads to the formation of products that can impair the organoleptic quality of the phospholipids. These byproducts can impart musty odors to the product and also a pungent aftertaste.

What is needed is an improved process for effectively recovering polar lipids and phospholipids rich in omega-3 and omega-6 HUFAs from biomaterials that enables the use of these fatty acid in food, nutritional supplement, pharmaceutical and cosmetic applications. Furthermore the fractions are needed as an ingredient in feed for companion animals and in aquaculture.

### SUMMARY OF THE INVENTION

In accordance with the present invention, an improved process is provided for recovering polar lipids enriched in omega-3 and/or omega-6 HUFAs from native biomaterials such as seeds and microorganisms and the use thereof.

In one embodiment of the present invention, a method is provided for providing a human, animal or aquaculture organism diet supplement enriched with at least one of eicosapentaenoic acid (EPA), docosahexaenoic acid (DHA), docosapentaenoic acid (n-3)(DPAn-3), eicosatetraenoic acid (n-3), docosapentaenoic acid (n-6)(DPAn-6) or arachidonic acid (ARA). The method includes the steps of producing a polar lipid-rich fraction enriched with at least one of EPA, DHA, DPA(n-3), DPA(n-6), eicosatetraenoic acid and ARA from genetically modified seeds or marine animals; and providing the polar lipid-rich fraction enriched with at least one of EPA, DHA, DPA(n-3), DPA(n-6), eicosatetraenoic acid and ARA in a form consumable or usable by humans or animals. Preferably, the animal is a companion animal.

In another embodiment of the present invention, a method is provided for treating a deficiency in at least one of EPA, DHA, DPA(n-3), DPA(n-6), eicosatetraenoic acid or ARA. The method includes the steps of producing a polar lipid-rich fraction enriched with at least one of EPA, DHA, DPA(n-3), DPA(n-6), eicosatetraenoic acid and ARA from microbes, genetically modified seeds or marine animals; and providing the polar lipid-rich fraction enriched with at least one of EPA, DHA, DPA(n-3), DPA(n-6), eicosatetraenoic acid and ARA to treat the deficiency. The deficiency can result in an inflammatory condition, an immune system imbalance, a cardiovascular disease, a developmental deficit related to nervous system development, a woman's health condition or an infant's health condition.

In another embodiment of the present invention, a method is provided for treating a chronic inflammatory disease state of the lung. The method includes the steps of producing a purified phospholipid fraction enriched with at least one of EPA, DHA, DPA(n-3), DPA(n-6), eicosatetraenoic acid and ARA from microbes, genetically modified seeds or marine animals; blending the phospholipid fraction enriched with at least one of EPA, DHA, DPA(n-3), DPA(n-6), eicosatetraenoic acid and ARA with at least one of EPA-, GLA- or SDA-rich oils; and producing an aerosol comprising the blend for the treatment of the disease states. The chronic inflammatory disease state of the lung can result in chronic obstructive pulmonary disease (COPD), asthma or cystic fibrosis.

In another embodiment of the present invention, a method is provided for the treatment of skin lesions, induced burn, UV-irradiation or other skin disorders. The method includes the steps of producing a purified phospholipid fraction enriched with at least one of EPA, DHA, DPA(n-3), DPA(n-6), eicosatetraenoic acid and ARA from microbes, genetically modified seeds or marine animals; blending the phospholipid fraction enriched with at least one of EPA, DHA, DPA(n-3), DPA(n-6), eicosatetraenoic acid and ARA with at least one EPA-, GLA- or SDA-rich oil; and producing a lotion or cream for the treatment of the skin disorders.

In another embodiment of the present invention, a method is provided for treating cachexia and severe fat malabsorption. The method includes the steps of producing a purified phospholipid enriched with at least one of EPA, DHA, DPA(n-3), DPA(n-6), eicosatetraenoic acid and ARA; blending the purified phospholipid enriched with at least one of EPA, DHA, DPA(n-3), DPA(n-6), eicosatetraenoic acid and ARA with at least one other purified phospholipid; blending the purified phospholipid enriched with at least one of EPA, DHA, DPA(n-3), DPA(n-6), eicosatetraenoic acid and ARA with at least one DHA, EPA, GLA- or SDA-rich oil; and producing a liquid or dried dietetic product for the treatment of the disease states. The cachexia or severe fat malabsorption can be a result of cancer or Crohn's disease. Preferably, the at least one other purified phospholipid is obtained from the group consisting of soy, rape seed, evening primrose, safflower, sunflower, canola, peanut, egg and mixtures thereof.

In another embodiment of the present invention, a method is provided for the treatment of *H. pylori*-infection of gastrointestinal tract. The method includes the steps of producing a purified phospholipid fraction enriched with at least one of EPA, DHA, DPA(n-3), DPA(n-6), eicosatetraenoic acid and ARA from microbes, genetically modified seeds or marine animals; blending the phospholipid fraction enriched with at least one of EPA, DHA, DPA(n-3), DPA(n-6), eicosatetraenoic acid and ARA with at least one EPA-, GLA- or SDA-rich oil; and producing a fat emulsion or a dietetic product for the treatment of the disease.

In another embodiment of the present invention, a method is provided for providing a fat blend enriched with at least one of EPA, DHA, DPA(n-3), DPA(n-6), eicosatetraenoic acid or ARA. The method includes the steps of extracting a polar lipid-rich fraction enriched with at least one of EPA, DHA, DPA(n-3), DPA(n-6), eicosatetraenoic acid and ARA from microbes, genetically modified seeds or marine animals; and mixing the polar lipid-rich fraction enriched with at least one of EPA, DHA, DPA(n-3), DPA(n-6), eicosatetraenoic acid and ARA with another oil. Preferably, the another oil is selected from the group consisting of fish oil, microbial oil, vegetable oil, GLA-containing oil, SDA-containing oil or mixtures thereof.

In another embodiment of the present invention, a method is provided for providing a blend of polar lipids enriched with at least one of EPA, DHA, DPA(n-3), DPA(n-6), eicosatetraenoic acid or ARA. The method includes the steps of extracting a polar lipid-rich fraction enriched with at least one of EPA, DHA, DPA(n-3), DPA(n-6), eicosatetraenoic acid and ARA from microbes, genetically modified seeds or marine animals; and mixing the polar lipid-rich fraction enriched with at least one of EPA, DHA, DPA(n-3), DPA(n-6), eicosatetraenoic acid and ARA with another polar lipid. Preferably, the another polar lipid is selected from the group consisting of soy polar lipids, rapeseed polar lipids, sunflower polar lipids, safflower polar lipids, canola polar lipids, peanut polar lipids or egg yolk polar lipids and mixtures thereof.

In another embodiment of the present invention, a fat blend is provided enriched with at least one of EPA, DHA, DPA(n-3), DPA(n-6), eicosatetraenoic acid or ARA. The fat blend includes a polar lipid-rich fraction enriched with at least one of EPA, DHA, DPA(n-3), DPA(n-6), eicosatetraenoic acid and ARA from microbes, genetically modified seeds or marine animals; and another oil. Preferably, the another oil is selected from the group consisting of fish oil, microbial oil, vegetable oil, GLA-containing oil, SDA-containing oil and mixtures thereof.

In another embodiment of the present invention, a method is provided for producing a blend of polar lipids enriched with at least one of EPA, DHA, DPA(n-3), DPA(n-6), eicosatetraenoic acid or ARA. The method includes the steps of extracting an EPA, DHA, DPA(n-3), DPA(n-6), eicosatetraenoic acid or ARA-enriched polar lipid-rich fraction from microbes, seeds or marine animals; and mixing the EPA, DHA, DPA(n-3), DPA(n-6), eicosatetraenoic acid or ARA-enriched polar lipid-rich fraction with another polar lipid. Preferably, the another polar lipid is selected from the group consisting of soy polar lipids, rapeseed polar lipids, sunflower polar lipids, safflower polar lipids, canola polar lipids, peanut polar lipids or egg yolk polar lipids and mixtures thereof.

In another embodiment of the present invention, purified phospholipids enriched with at least one of EPA, DHA, DPA(n-3), DPA(n-6), eicosatetraenoic acid or ARA derived from polar lipid-rich fraction extracted from genetically modified seeds or marine animals are provided. Preferably, the EPA, DHA, DPA(n-3), DPA(n-6), eicosatetraenoic acid or ARA-enriched phospholipid-fraction is in a form consumable or usable by humans or animals.

In another embodiment of the present invention, a method is provided for providing a human, animal or aquaculture organism diet supplement enriched with at least one of eicosapentaenoic acid (EPA), docosapentaenoic acid (n-3)(DPAn-3), eicosatetraenoic acid (n-3) or docosapentaenoic acid (n-6)(DPAn-6). The method includes the steps of producing a polar lipid-rich fraction enriched with at least one of EPA, DPA(n-3), DPA(n-6) and eicosatetraenoic acid from microbes, genetically modified seeds or marine animals; and providing the polar lipid-rich fraction enriched with at least one of EPA, DPA(n-3), DPA(n-6) and eicosatetraenoic acid in a form consumable or usable by humans or animals.

The polar lipid-rich fraction of the methods or products of can be provided as an ingredient of dietetic, pharmaceutical and cosmetic applications.

The fat blend of the methods or products of the present invention can be provided as an ingredient of dietetic, pharmaceutical and cosmetic applications.

The blend of polar lipids of the methods or products of the present invention can be provided as an ingredient of dietetic, pharmaceutical and cosmetic applications.

The purified phospholipids of the methods or products of the present invention can be provided as an ingredient of dietetic, pharmaceutical and cosmetic applications.

Preferably, the marine animals of the methods and products of the present invention are fish, squid, mollusks or shrimp. Preferably, the marine animals are fish or fish eggs from the group including salmon, tuna, haddock, sardines, mackerel, or menhaden.

Preferably, the microbes of the methods and products of the present invention are selected from fungi, microalgae, protozoa or bacteria. More preferably, microbes are selected from the Stramenopiles, Thraustochytriales, Chrysophyceae, Xanthophyceae, Bacillariophyceae, Dinophyceae, Phaeophyceae, Rhodophyceae, Chlorophyceae, Euglenophyceae, Cryptophyceae, Oomycetes, Chytridomycetes, or Zygomycetes. More preferably, the microbes are selected from the group of genera consisting of Mortierella, Mucor, Phycomyces, Rhizopus, Pythium, Ochramonas, Nitzschia, Phaeodactylum, Skeletonema, Fucus, Laminaria, Platymonas, Achyla, Phytophera, Schizochytrium, Thraustochytrium, or Crypthecodinium.

Preferably, the EPA, DHA, DPA(n-3), DPA(n-6), eicosatetraenoic acid, ARA or mixture thereof employed in the methods and products of the present invention makes up at least two weight percent of the total fatty acids of the polar lipid fraction.

Preferably, the EPA, DHA, DPA(n-3), DPA(n-6), eicosatetraenoic acid, ARA or mixture thereof employed in the methods and products at the present invention makes up at least five weight percent of the total fatty acids of the polar lipid fraction.

Preferably, the plant seeds or microbes employed in the methods and products to the present invention have been genetically modified to increase their n-3 or n-6 HUFA content.

Preferably, the seeds or microbes used in the methods and products of the present invention have been genetically modified to increase the production of at least one of EPA, DHA, DPA(n-3), DPA(n-6), eicosatetraenoic acid or ARA.

Preferably, the seeds employed in the methods and products of the present invention are selected from the group consisting of canola, rapeseed, linseed, flaxseed, sunflower, safflower, peanuts, soybeans or corn. Preferably, the polar lipid-rich fraction is extracted from the seeds and microbes using alcohol.

In an alternative embodiment of the present invention, the polar lipid-rich fraction is derived as a by-product (e.g., by de-gumming) of oil extraction from the seeds using hexane and other non-polar solvents.

Preferably, the polar lipid-rich fraction used in the methods and products of the present invention is extracted from the seeds and microbes by use of gravity or centrifugal extraction technology.

### DETAILED DESCRIPTION OF THE INVENTION

Because of their bipolar nature, polar lipids (including phospholipids) are of significant commercial interest as wetting and emulsifying agents. These properties may also help make HUFAs in the phospholipids more bioavailable, in addition to enhancing their stability. These properties make phospholipids ideal forms of ingredients for use in nutritional supplements, food, infant formula, pharmaceutical, and cosmetic applications. Dietary benefits of phospholipids include both improved absorption and improved incorporation. Phospholipids also have a broad range of functionality in the body in that they are important cell membrane constituents, they are good emulsifiers, they can act as intestinal surfactants, serve as a choline source and as a source of HUFAs.

EPA, DHA, and ARA are normally produced for the nutritional supplement market and food uses through the extraction (by cooking) of fish (EPA and DHA) such as menhaden, tuna or salmon, or hexane extraction of fungal biomass (ARA) from the genus Mortierella. The phospholipids in both processes are removed in a later degumming step that produces a waste material comprising a complex mixture of neutral lipids, sterols, glucosides and phospholipids. This material is normally sold to the domestic animal feed industry to dispose of it.

Besides fish and fungal biomass, there are microbial sources of DHA and DPA(n-6). For the present invention, useful microbes can be selected from fungi, microalgae, protozoa or bacteria. These organisms can be selected from the groups including the Stramenopiles, Thraustochytriales, Chrysophyceae, Xanthophyceae, Bacillariophyceae, Dinophyceae, Phaeophyceae, Rhodophyceae, Chlorophyceae, Euglenophyceae, Cryptophyceae, Oomycetes, Chytridomycetes, or Zygomycetes. Useful microbes can also be selected from the group of genera consisting of Mortierella, Mucor, Phycomyces, Rhizopus, Pythium, Ochromonas, Nitzschia, Phaeodactylum, Skeletonema, Fucus, Platymonas, Achyla, Phytophera, Schizochytrium, Thraustochytrium, or Crypthecodinium. Microorganisms are good sources of phospholipids because they can be grown in culture in a manner that optimizes phospholipid production and minimizes triglyceride (oil) production. On the other hand the methods used in this invention allow both oil and phospholipids to be recovered separately in forms that can be used directly in food, feed, nutritional supplements, cosmetic or pharmaceutical application.

DHA, EPA and ARA phospholipids can be recovered from fish, microalgae, or fungi through the degumming process described above. However as noted this produces a complex material containing many other compounds including neutral lipids, sterols, glucosides, etc

A preferred embodiment of the present invention is to use alcohol and centrifugation to recover the omega-3 and/or omega-6 HUFA-rich phospholipids. Preferred methods for this recovery are described in the following references, which are incorporated by reference herein in their entirety:
i. PCT Application Serial No. PCT/US01/12047, entitled "Method for the Fractionation of Oil and Polar Lipid-Containing Native Raw Materials" filed April 12, 2001;
ii. PCT Application Serial No. PCT/US01/12049, entitled "Method For The Fractionation Of Oil And Polar Lipid-Containing Native Raw Materials Using Water-Soluble Organic Solvent And Centrifugation" filed April 12, 2001.

Once the omega-3 and/omega-6 rich phospholipid fractions have been extracted by these preferred processes, they can be used directly as ingredients or they can be purified further and even separated into phospholipid classes by well-known techniques such as different forms of chromatography, molecular distillation, and special refining techniques. Polar lipid groups phosphatidylcholine, phosphatidylserine, phosphatidylethanolamine, phosphatidylinositol, and sphingolipids. The phospholipid rich polar lipids or the purified phospholipid rich fractions can also be mixed with another lipid or oil such as fish lipids, microbial lipids, vegetable lipids, GLA-containing lipids, SDA-containing lipids and mixtures thereof, or be mixed with another phospholipid fraction (lecithin) such as soy or egg yolk lecithin, sunflower lecithin, peanut lecithin or mixtures thereof prior to use as a nutritional supplement, feed or food ingredient. These mixtures of phospholipids can also be incorporated into creams or lotions for topical applications (e.g. treating of skin conditions) or skin lesions induced by burns, UV-irradiation or other skin damaging processes. The mixtures can also be processed to produce a liquid or spray-dried dietetic product or fat emulsion for treating cachexia and severe fat malabsorption or for treatment of *H. pylori* infection of the gastrointestinal tract or incorporated into aerosol sprays for treating chronic inflammatory disease states of the lung (e.g., COPD, asthma, cystic fibrosis).

Advantages of the present invention including providing omega-3 and/or omega-6 HUFAs in a more bioactive and functional form (phospholipid) than the triglyceride form and include a better process (e.g., a) no need for heat treatment; b) no use of toxic solvents (like hexane) and c) no artifacts and off-flavors due to the use of acetone) for recovering these phospholipids from oilseeds and microbes. The EPA, DHA, DPA(n-3), DPA(n-6), eicosatetraenoic acid and/or ARA content of the total fatty acids of the polar lipid fractions and comprise at least two weight percent of the total fatty acids of the polar lipid fraction, more preferably greater than 5 weight percent, more preferably greater than 10 weight percent, and most preferably greater than 20 weight percent of total fatty acids in the polar lipid fraction.

### EXAMPLE

### Example 1

Phospholipids were extracted from salmon, salmon roe, Black tiger prawns, squid, Schizochytrium sp, and the total fatty acid content of the phospholipids was determined by gas chromatography. The results are presented in Table 1. As can be observed the phospholipid fraction of these biomaterials can be used to deliver omega-3 and/or omega-6 HUFAs and in this form these bioactive fatty acids should be more stable, more bioavailable, and more functional.

**Table 1. Total fatty acid content of phospholipids extracted from 4 types marine animal products and forma microbial source.**

| | | **Salmon** | **Salmon roe** | **Tiger Prawn** | **Squid** | **Schizochytrium** |
|---|---|---|---|---|---|---|
| COMPOUND | | **PL's** | **PL's** | **PL's** | **PL's** | **PL's** |
| | | % TFA | % TFA | %TFA | % TFA | % TFA |
| MYRISTATE | C14:0 | 2.99 | 4.58 | 1.14 | 1.87 | 2.66 |
| MYRISTOLEATE | C14:1 | 0,00 | 0,00 | 0.05 | 0.03 | 0,00 |
| PALMITATE | C16:0 | 26.48 | 29.661 | 25.44 | 30.37 | 28.25 |
| PALMITOLEATE | C16:1 | 2.86 | 4.38 | 1.55 | 0.23 | 0.62 |
| STEARATE | C18:0 | 5.74 | 6.19 | 13.59 | 4.41 | 1.75 |
| OLEATE | C18:1 | 9.74 | 13.36 | 13.33 | 1.86 | 6.99 |
| LINOLEATE | C18:2n6 | 3.14 | 1.05 | 12.51 | 0.32 | 2.46 |
| GAMMA LINOLENATE | C18:3n6 | 0.00 | 0.00 | 0.00 | 0.00 | 1.09 |
| ARACHIDATE | C20:0 | 0.00 | 0.00 | 0.74 | 0.13 | 0,00 |
| LINOLENATE | C18:3n3 | 0.49 | 0.22 | 0.54 | 0.05 | 0.00 |
| OCTADECATETRAENOATE | C18:4 | 0.50 | 0.33 | 0.08 | 0.07 | 0.00 |
| EICOSENOATE-11 | C20:1 | 0,75 | 1.07 | 0.85 | 5.54 | 0.00 |
| EICOSADIENOATE-11,14 | C20:2 | 0,00 | 0.00 | 0.56 | 0.34 | 0,00 |
| BEHENATE | C22:0 | 0,00 | 0,00 | 1.35 | 0.08 | 0,00 |
| EICOSATRIENOATE | C20:3n3 | 1.01 | 3.58 | 0.07 | 0.42 | 1.61 |
| ARACHIDONATE | C20:4 n6 | 0,89 | 0.56 | 5.54 | 1.70 | 1.18 |
| ERUCATE | C22:1 | 0,21 | 0,00 | 0.04 | 1.41 | 0.00 |
| EICOSAPENTAENOATE | C20:5n3 | 9.50 | 9.39 | 8.47 | 14.71 | 5.11 |
| LIGNOCERATE | C24:0 | 0,00 | 0,00 | 0.56 | 0.00 | 0.00 |
| NERVONATE | C24:1 | 1.61 | 1.65 | 0.73 | 0.82 | 0,00 |
| DOCOSAPENTAENOATE n-6 | C22:5n6 | 0,00 | 0,00 | 0.45 | 0.22 | 17.68 |
| DOCOSAPENTAENOATE n-3 | C22:5n3 | 3.16 | 4.40 | 0.55 | 0.44 | 0,00 |
| DOCOSAHEXAENOATE | C22:6n3 | 30.92 | 19.50 | 11.86 | 35.54 | 30.61 |
| | | 100,00 | 100,00 | 100.00 | 100,00 | 100,00 |

The present invention, in various embodiments, includes components, methods, processes, systems and/or apparatus substantially as depicted and described herein, including various embodiments, subcombinations, and subsets thereof. Those of skill in the art will understand how to make and use the present invention after understanding the present disclosure. The present invention, in various embodiments, includes providing devices and processes in the absence of items not depicted and/or described herein or in various embodiments hereof, including in the absence of such items as may have been used in previous devices or processes, e.g., for improving performance, achieving ease and/or reducing cost of implementation.

The foregoing discussion of the invention has been presented for purposes of illustration and description. The foregoing is not intended to limit the invention to the form or forms disclosed herein. Although the description of the invention has included description of one or more embodiments and certain variations and modifications, other variations and modifications are within the scope of the invention, e.g., as may be within the skill and knowledge of those in the art, after understanding the present disclosure. It is intended to obtain rights which include alternative embodiments to the extent permitted, including alternate, interchangeable and/or equivalent structures, functions, ranges or steps to those Claimed, whether or not such alternate, interchangeable and/or equivalent structures, functions, ranges or steps are disclosed herein, and without intending to publicly dedicate any patentable subject matter.

Various preferred features and embodiments of the present invention will now be described with reference to the following numbered paragraphs (paras).
1. A method for providing a human, animal or aquaculture organism diet supplement enriched with at least one of eicosapentaenoic acid (EPA), docosahexaenoic acid (DHA), docosapentaenoic acid (n-3)(DPAn-3), eicosatetraenoic acid (n-3), docosapentaenoic acid (n-6)(DPAn-6) or arachidonic acid (ARA) comprising the steps:
   (a) producing a polar lipid-rich fraction enriched with at least one of EPA, DHA, DPA(n-3), DPA(n-6), eicosatetraenoic acid and ARA from genetically modified seeds or marine animals; and
   (b) providing said polar lipid-rich fraction enriched with at least one of EPA, DHA, DPA(n-3), DPA(n-6), eicosatetraenoic acid and ARA in a form consumable or usable by humans or animals.
2. The method of para 1, wherein the animal is a companion animal.
3. A method for treating a deficiency in at least one of EPA, DHA, DPA(n-3), DPA(n-6), eicosatetraenoic acid or ARA comprising the steps:
   (a) producing a polar lipid-rich fraction enriched with at least one of EPA, DHA, DPA(n-3), DPA(n-6), eicosatetraenoic acid and ARA from microbes, genetically modified seeds or marine animals; and
   (b) providing said polar lipid-rich fraction enriched with at least one of EPA, DHA, DPA(n-3), DPA(n-6), eicosatetraenoic acid and ARA to treat said deficiency.
4. The method of para 3, wherein said deficiency results in an inflammatory condition, an immune system imbalance, a cardiovascular disease, a developmental deficit related to nervous system development, a woman's health condition or an infant's health condition.
5. A method for treating a chronic inflammatory disease state of the lung comprising the steps:
   (a) producing a purified phospholipid fraction enriched with at least one of EPA, DHA, DPA(n-3), DPA(n-6), eicosatetraenoic acid and ARA from microbes, genetically modified seeds or marine animals;
   (b) blending said phospholipid fraction enriched with at least one of EPA, DHA, DPA(n-3), DPA(n-6), eicosatetraenoic acid and ARA with at least one of EPA-, GLA- or SDA-rich oils; and
   (c) producing an aerosol comprising the blend of step (b) for the treatment of said disease states.
6. The method of para 5, wherein the chronic inflammatory disease state of the lung is COPD, asthma or cystic fibrosis.
7. A method for the treatment of skin lesions, induced burn, UV-irradiation or other skin disorders comprising the steps:
   (a) producing a purified phospholipid fraction enriched with at least one of EPA, DHA, DPA(n-3), DPA(n-6), eicosatetraenoic acid and ARA from microbes, genetically modified seeds or marine animals;
   (b) blending said phospholipid fraction enriched with at least one of EPA, DHA, DPA(n-3), DPA(n-6), eicosatetraenoic acid and ARA with at least one EPA-, GLA- or SDA-rich oil; and
   (c) producing a lotion or cream for the treatment of said skin disorders.
8. A method for treating cachexia or fat malabsorption comprising the steps:
   (a) producing a purified phospholipid enriched with at least one of EPA, DHA, DPA(n-3), DPA(n-6), eicosatetraenoic acid and ARA;
   (b) blending said purified phospholipid enriched with at least one of EPA, DHA, DPA(n-3), DPA(n-6), eicosatetraenoic acid and ARA with at least one other purified phospholipid;
   (c) blending said purified phospholipid enriched with at least one of EPA, DHA, DPA(n-3), DPA(n-6), eicosatetraenoic acid and ARA with at least one DHA, EPA, GLA- or SDA-rich oil; and
   (d) producing a liquid or dried dietetic product for the treatment of said disease states.
9. The method of para 8, wherein the cachexia or fat malabsorption is a result of cancer or Crohn's disease.
10. The method of para 8, wherein the at least one other purified phospholipid is obtained from the group consisting of soy, rape seed, evening primrose, safflower, sunflower, canola, peanut, egg and mixtures thereof.
11. A method for the treatment of *H. pylori*-infection of gastrointestinal tract comprising the steps:
   (a) producing a purified phospholipid fraction enriched with at least one of EPA, DHA, DPA(n-3), DPA(n-6), eicosatetraenoic acid and ARA from microbes, genetically modified seeds or marine animals;
   (b) blending said phospholipid fraction enriched with at least one of EPA, DHA, DPA(n-3), DPA(n-6), eicosatetraenoic acid and ARA with at least one EPA-, GLA- or SDA-rich oil; and
   (c) producing a fat emulsion or a dietetic product for the treatment of said disease.
12. A method for providing a fat blend enriched with at least one of EPA, DHA, DPA(n-3), DPA(n-6), eicosatetraenoic acid or ARA comprising the steps:
   (a) extracting a polar lipid-rich fraction enriched with at least one of EPA, DHA, DPA(n-3), DPA(n-6), eicosatetraenoic acid and ARA from microbes, genetically modified seeds or marine animals; and
   (b) mixing said polar lipid-rich fraction enriched with at least one of EPA, DHA, DPA(n-3), DPA(n-6), eicosatetraenoic acid and ARA with another oil.
13. The method of para 12, wherein said another oil is selected from the group consisting of fish oil, microbial oil, vegetable oil, GLA-containing oil, SDA-containing oil or mixtures thereof.
14. A method for providing a blend of polar lipids enriched with at least one of EPA, DHA, DPA(n-3), DPA(n-6), eicosatetraenoic acid or ARA comprising the steps:
   (a) extracting a polar lipid-rich fraction enriched with at least one of EPA, DHA, DPA(n-3), DPA(n-6), eicosatetraenoic acid and ARA from microbes, genetically modified seeds or marine animals; and
   (b) mixing said polar lipid-rich fraction enriched with at least one of EPA, DHA, DPA(n-3), DPA(n-6), eicosatetraenoic acid and ARA with another polar lipid.
15. The method of para 14, wherein said another polar lipid is selected from the group consisting of soy polar lipids, rape seed polar lipids, sunflower polar lipids, safflower polar lipids, canola polar lipids, peanut polar lipids or egg yolk polar lipids and mixtures thereof.
16. A fat blend enriched with at least one of EPA, DHA, DPA(n-3), DPA(n-6), eicosatetraenoic acid or ARA comprising:
   (a) a polar lipid-rich fraction enriched with at least one of EPA, DHA, DPA(n-3), DPA(n-6), eicosatetraenoic acid and ARA from microbes, genetically modified seeds or marine animals; and
   (b) another oil.
17. The oil of para 16, wherein said another oil is selected from the group consisting of fish oil, microbial oil, vegetable oil, GLA-containing oil, SDA-containing oil and mixtures thereof.
18. A blend of polar lipids enriched with at least one of EPA, DHA, DPA(n-3), DPA(n-6), eicosatetraenoic acid or ARA comprising the steps:
   (a) extracting a EPA, DHA, DPA(n-3), DPA(n-6), eicosatetraenoic acid or ARA-enriched polar lipid-rich fraction from microbes, seeds or marine animals; and
   (b) mixing said EPA, DHA, DPA(n-3), DPA(n-6), eicosatetraenoic acid or ARA-enriched polar lipid-rich fraction with another polar lipid.
19. The method of para 18, wherein said another polar lipid is selected from the group consisting of soy polar lipids, rape seed polar lipids, sunflower polar lipids, safflower polar lipids, canola polar lipids, peanut polar lipids or egg yolk polar lipids and mixtures thereof.
20. Purified phospholipids enriched with at least one EPA, DHA, DPA(n-3), DPA(n-6), eicosatetraenoic acid or ARA derived from polar lipid-rich fraction extracted from genetically modified seeds or marine animals.
21. The purified phospholipids of para 20, wherein said EPA, DHA, DPA(n-3), DPA(n-6), eicosatetraenoic acid or ARA-enriched phospholipid-fraction is in a form consumable or usable by humans or animals.
22. A method for providing a human, animal or aquaculture organism diet supplement enriched with at least one of eicosapentaenoic acid (EPA), docosapentaenoic acid (n-3)(DPAn-3), eicosatetraenoic acid (n-3) or docosapentaenoic acid (n-6)(DPAn-6) comprising the steps:
   (a) producing a polar lipid-rich fraction enriched with at least one of EPA, DPA(n-3), DPA(n-6) and eicosatetraenoic acid from microbes, genetically modified seeds or marine animals; and
   (b) providing the polar lipid-rich fraction enriched with at least one of EPA, DPA(n-3), DPA(n-6) and eicosatetraenoic acid in a form consumable or usable by humans or animals.
23. A polar lipid-rich fraction of the method or product of any preceding para as an ingredient of dietetic, pharmaceutical or cosmetic applications.
24. A fat blend of the method or product of any preceding para as an ingredient of dietetic, pharmaceutical or cosmetic applications.
25. A blend of polar lipids of the method or product of any preceding para as an ingredient of dietetic, pharmaceutical or cosmetic applications.
26. Purified phospholipids of the method or product of any preceding para as an ingredient of dietetic, pharmaceutical or cosmetic applications.
27. The method or product of any preceding para, wherein said marine animals are fish, squid, mollusks or shrimp.
28. The method or product of any preceding para, wherein said marine animals are fish or fish eggs from the group including salmon, tuna, haddock, sardines, mackerel, or menhaden.
29. The method or product of any preceding para, wherein said microbes are selected from fungi, microalgae, protozoa or bacteria.
30. The method or product of any preceding para, wherein said microbes are selected from the Stramenopiles, Thraustochytriales, Chrysophyceae, Xanthophyceae, Bacillariophyceae, Dinophyceae, Phaeophyceae, Rhodophyceae, Chlorophyceae, Euglenophyceae, Cryptophyceae, Oomycetes, Chytridomycetes, or Zygomycetes.
31. The method or product of any preceding para, wherein said microbes are selected from the group of genera consisting of Mortierella, Mucor, Phycomyces, Rhizopus, Pythium, Ochromonas, Nitzschia, Phaeodactylum, Skeletonema, Fucus, Laminaria, Platymonas, Achyla, Phytophera, Schizochytrium, Thraustochytrium, or Crypthecodinium.
32. The method of or product of any preceding para, wherein EPA, DHA, DPA(n-3), DPA(n-6), eicosatetraenoic acid, ARA or mixture thereof comprises at least two weight percent of the total fatty acids of the polar lipid fraction.
33. The method or product of any preceding para, wherein EPA, DHA, DPA(n-3), DPA(n-6), eicosatetraenoic acid, ARA or mixture thereof comprises at least five weight percent of the total fatty acids of the polar lipid fraction.
34. The method or product of any preceding para, wherein said plant seeds or microbes have been genetically modified to increase their n-3 or n-6 HUFA content.
35. The method or product of any preceding para, wherein said seeds or microbes have been genetically modified to increase the production of at least one of EPA, DHA, DPA(n-3), DPA(n-6), eicosatetraenoic acid or ARA.
36. The method or product of any preceding para, wherein said seeds are selected from the group consisting of canola, rape seed, linseed, flaxseed, sunflower, safflower, peanuts, soybeans or corn.
37. The method or product of any preceding para, wherein said polar lipid-rich fraction is extracted from said seeds and microbes using alcohol.
38. The method or product of any preceding para, wherein said polar lipid-rich fraction is derived as a by-product of oil extraction from said seeds using hexane or other non-polar solvent.
39. The method or product of any preceding para, wherein said polar lipid-rich fraction is extracted from said seeds and microbes by use of gravity or centrifugal extraction technology.

## Claims

1. A composition comprising:
(a) a polar lipid-rich fraction enriched with at least one of eicosapentaenoic acid (EPA), docosahexaenoic acid (DHA), docosapentaenoic acid (n-3) (DPA(n-3)), docosapentaenoic acid (n-6) (DPA(n-6)), eicosatetraenoic acid, and arachidonic acid (ARA), wherein the polar lipid-rich fraction is extracted from microbes or from genetically modified seeds; and
(b) an oil.

2. The composition of claim 1, wherein the polar lipid-rich fraction is extracted from genetically modified seeds.

3. The composition of claim 2, wherein the genetically modified seeds are selected from the group consisting of: canola, rape seed, linseed, flaxseed, sunflower, safflower, peanuts, soybeans, and corn.

4. The composition of claim 1, wherein the polar lipid-rich fraction is extracted from microbes.

5. The composition of claim 4, wherein the microbes are selected from the group of genera consisting of: *Mortierella, Mucor, Phycomyces, Rhizopus, Pythium, Ochromonas, Nitzschia, Phaeodactylum, Skeletonema, Laminaria, Platymonas, Achyla, Phytophera, Schizochytrium, Thraustochytrium,* and *Crypthecodinium.*

6. The composition of any preceding claim, wherein the oil is selected from the group consisting of: fish oil, microbial oil, vegetable oil, and mixtures thereof.

7. The composition of any preceding claim, wherein the oil is selected from the group consisting of: eicosapentaenoic acid (EPA)-containing oil, gamma-linolenic (GLA)-containing oil, stearidonic acid (SDA)-containing oil, and mixtures thereof.

8. The composition of any preceding claim, wherein the EPA, DHA, DPA(n-3), DPA(n-6), eicosatetraenoic acid, or ARA comprises at least two weight percent of the total fatty acids of the polar lipid fraction.

9. The composition of claim 8, wherein the EPA, DHA, DPA(n-3), DPA(n-6), eicosatetraenoic acid, or ARA comprises at least five weight percent of the total fatty acids of the polar lipid fraction.

10. The composition of any preceding claim, wherein the genetically modified seeds or microbes have been genetically modified to increase their n-3 or n-6 HUFA content.

11. The composition of any preceding claim, wherein the genetically modified seeds or microbes have been genetically modified to increase the production of at least one of EPA, DHA, DPA(n-3), DPA(n-6), eicosatetraenoic acid, and ARA.

12. The composition of any preceding claim, wherein the polar lipid-rich fraction is extracted using alcohol.

13. The composition of any preceding claim, wherein the polar lipid-rich fraction is derived as a by-product of oil extraction using hexane or other non-polar solvent.

14. The composition of any preceding claim, wherein the polar lipid-rich fraction is extracted by use of gravity or centrifugal extraction technology.

15. A method for providing the composition of any preceding claim, comprising:
(a) extracting a polar lipid-rich fraction from microbes or genetically modified seeds; and
(b) mixing the polar lipid-rich fraction with an oil.

16. A composition of any one of claims 1 to 14 for use in treating a condition selected from the group consisting of: an inflammatory condition, an immune system imbalance, a cardiovascular disease, a developmental deficit related to nervous system development, a woman's health condition, and an infant's health condition.

17. The composition of claim 16, wherein the condition results from a deficiency in at least one of EPA, DHA, DPA(n-3), DPA(n-6), eicosatetraenoic acid, and ARA.
